# EUROPEAN PATENT APPLICATION

(11) **EP 2 380 990 A1**
(43) Date of publication of application: **26.10.2011**
(21) Application number: 10160472.6
(22) Date of filing: 20.04.2010
(51) Int. Cl.: C12Q 1/34, C12Q 1/42, C12Q 1/44, B22F 9/00, C12P 3/00

(54) **Analytical applications of enzymatic growth of fluorescent quantum dots**

(71) Applicant: Asociación Centro de Investigación Cooperativa en Biomateriales - CIC biomaGUNE, 2008 San Sebastián (ES)
(72) Inventor: PAVLOV, Valery, 428022 Cheboksary (RU); SAA PEÑA, Laura, 20016 San Sebastian (ES); VIREL SÁNCHEZ, Ana, 20009 San Sebastian (ES)
(74) Representative: ZBM Patents

(57) **Abstract**

Methods for the detection/quantification of the enzymatic activity of hydrolases in a sample, wherein the hydrolase catalyses a hydrolysis reaction of a substrate which yields either H₂S or a thiol-containing organic compound that produces a decomposition reaction which generates H₂S, and methods for the detection/quantification of their substrates in a sample, as well as a method for the preparation of fluorescent CdS quantum dots, the preparation method, comprising first carrying out a enzymatic reaction catalysed by the hydrolase wherein the hydrolase catalyses the hydrolysis reaction of a substrate which yields either H₂S or a thiol-containing organic compound that produces a decomposition reaction which generates H₂S, and then reacting the resulting H₂S with a salt of Cd⁺², thereby fluorescent CdS quantum dots are obtained.

## Description

### FIELD OF THE INVENTION

The present invention provides methods for the detection/quantification of the enzymatic activity of hydrolases and methods for the detection/quantification of their substrates based on the generation of fluorescent CdS quantum dots, as well as methods for the preparation of fluorescent CdS quantum dots.

### BACKGROUND ART

Metallic and semiconductor nanoparticles are extensively employed in bio analytical chemistry. Inorganic semiconductor nanoparticles can be photo excited to generate electron/hole couples which recombine to yield fluorescent emission of light. Their emission properties are a consequence of quantum effects, hence, they are frequently called quantum dots (QDs). The most important advantages of QDs over organic fluorophores are: (1) higher brightness, (2) reduced photobleaching and (3) longer lifetimes.

QDs have been generally used as fluorescent labels in biosensing, especially in assays based on detection of analytes by affinity binding. QDs have also been used as energy-transfer donors in assays based on fluorescence energy transfer (FRET) (Goldman 2005).

Most of the reported assays related to the enzymatic generation of nanoparticles are limited to biochemical growth of non-fluorescent metal nanoparticles (Xiao 2005). The main drawback of the systems is that only UV-visible spectroscopy can be utilised for the optical read out of the generated signal.

The other group of reported assays related to the enzymatic generation of nanoparticles relates to the production of CdS nanoparticles from the reduction of Cd²⁺ and SO₄²⁻ ions using sulphite reductase enzyme purified from Fusarium oxysporum (Ansary 2007). Said method relates to the *in vitro* production of CdS QDs, and its characterisation; it is not a method to detect enzymatic activity. Therefore, it is not a bioanalytical assay and the method has, hence, no analytical application.

The use of QDs in the field of bioanalytical analysis of enzymatic activities has so far been limited to FRET assays for the analysis of the enzymatic activity of a very limited number of enzymes, specifically proteases (Medintz 2006).

Among the enzymes whose enzymatic activity is detected daily for a wide number of applications are the hydrolases, such as the alkaline phosphatase (ALP) and the acetylcholine esterase (AChE).

In biochemistry, the hydrolase catalyses the hydrolysis of a chemical bond according to the following reaction:

A-B + H₂O → A-OH + B-H

Wherein A is an (in)organic moiety and B is H or an (in)organic moiety.

According to the Enzyme Commission (EC) the EC number of the hydrolases is EC 3. Hydrolases can be further classified into several subclasses, based on the bonds they act upon.

The detection and quantification of the enzymatic activity of hydrolases has a great interest in the fields of medicine (clinical diagnosis), plant pathology, industry (i.e. quality control check), etc. Among the hydrolases, the following ones are especially relevant:
1. ALP (EC 3.1.3.1) also known as phosphate-monoester phosphohydrolase (alkaline optimum). ALP is responsible for the dephosphorylation or removal of phosphate groups from many types of molecules, including nucleotides, proteins and alkaloids.

ALP finds a wide application in bioanalysis, especially as a label in Enzyme-Linked Immunosorbent Assays, also called ELISA (a well known biochemical technique used mainly in immunology to detect the presence of an antigen in a sample, wherein the detection antibody is linked to an enzyme, being that enzyme mainly ALP); Southern, Western and Northern blotting; and dot/slot blots. Another important application of ALP is the monitoring of pasteurisation in cow's milk: well pasteurised milk should not demonstrate any ALP activity due to the enzyme deactivation at elevated temperature. Finally, the measurement of ALP in human blood serum is also used in medicine, i.e. for the diagnosis of acute and chronic viral hepatitis and liver function test (i.e. cirrhosis). ALP is an enzyme in the cells lining the biliary ducts of the liver. ALP levels in plasma will rise with large bile duct obstruction, intrahepatic cholestasis or infiltrative diseases of the liver.

ALP activity is tested in the current state-of-the-art by chromogenic substances: p-nitrophenyl phosphate, 5-bromo-4-chloro-3-indolyl phosphate and 1-naphtyl phosphate disodium salt (Horwitz 1966). Fluorogenic substrates based on 4-methylumbelliferone have also been employed in the activity assay of ALP. In summary, the current analytical detection of ALP enzymatic activity is based on the detection using UV-visible spectroscopy. The disadvantages of the above methods include high costs, special storage conditions and maximum fluorescence of the reaction product at alkaline pH.
2. AChE, also known as acetylcholinesterase (EC 3.1.1.7). AChE catalyses the hydrolysis of the neurotransmitter acetylcholine into choline and acetic acid at the cholinergic synapses. Inhibitors of AChE such as nerve gases and pesticides are able to block enzymatic decomposition of acetylcholine causing fatal consequences. AChE inhibitors have been used clinically as Alzheimer's treatment and are the subject of great interest in various disease processes and treatment strategies (i.e. vitiligo). However, both environmental detection of AChE inhibitors and development of new modulators of AChE enzymatic activity as drugs have been hampered by the difficulty and complexity of the current assay methods.

The traditional assay for AChE activity involves colorimetric detection in a reaction employing Ellman's reagent. This assay is too slow and inadequately sensitive for use in high-throughput applications. Other alternative methods have been developed such as that based on the use of a thiol-specific electrocatalytic electrode (Mukherjee 2008). But they are too expensive, difficult to perform and of limited used.

Notwithstanding the current knowledge of detection systems based on the use of quantum dots, no advance has been done to develop new methods for the rapid and accurate detection/quantification of enzymatic activities of some hydrolases, such as ALP and AChE, based on them.

In summary, current state-of-the-art methods for the analytical detection of enzymatic activity of some hydrolases, i.e. ALP and AChE, have serious drawbacks as explained above. A bioanalytical enzymatic system producing fluorescent nanoparticles could employ a much more sensitive technique, such as fluorescence spectroscopy.

Therefore, a rapid and accurate analytical method to determine the enzymatic activity of some hydrolases is required. This suggests the need for a new, simplified and improved analytical method of enzymatic activities of some hydrolases.

### SUMMARY OF THE INVENTION

The present invention fulfils the above mentioned need, and further provides other related advantages.

The present invention encompasses useful methods in the quantification and detection of the enzymatic activity of some hydrolases based on the generation of fluorescent CdS QDS as well as methods for the preparation of fluorescent CdS QDs.

The present invention fulfils a long-felt need, namely, rapid, accurate and low cost methods for the detection of the enzymatic activity of some hydrolases.

The present invention is based on the unexpected inventors' finding that a reaction product generated by the enzymatic activity of some hydrolases yields fluorescent CdS QDs.

The present invention, hence, provides simple, inexpensive and novel methods for detecting enzymatic activity of a hydrolase in a sample wherein the hydrolase catalyses a hydrolysis reaction of a substrate which yields either H₂S or a thiol-containing organic compound that produces a decomposition reaction which generates H₂S, the method, comprising: (a) first adding the substrate to the sample and carrying out a reaction which generates H₂S, and then reacting the resulting H₂S with a salt of Cd⁺², thereby fluorescent CdS quantum dots are obtained; and (b) detecting the fluorescent CdS quantum dots of step a). The present invention also comprises said method wherein the detection of fluorescence indicates the detection of the hydrolase in the sample.

The present invention also provides a method of quantifying enzymatic activity of a hydrolase in a sample, wherein the hydrolase catalyses a hydrolysis reaction of a substrate which yields either H₂S or a thiol-containing organic compound that produces a decomposition reaction which generates H₂S, the method, comprising: (a) first adding the substrate to the sample and carrying out a reaction which generates H₂S, and then reacting the resulting H₂S with a salt of Cd⁺², thereby fluorescent CdS quantum dots are obtained; (b) measuring the fluorescence generated by the CdS quantum dots; (c) generating a calibration curve; and (d) extrapolating the concentration of the hydrolase in the sample using the calibration curve and the fluorescence measurement obtained for the sample.

Another object of the present invention is the provision of a method for detecting the substrate of a hydrolase in a sample, wherein the hydrolase catalyses a hydrolysis reaction of the substrate which yields either H₂S or a thiol-containing organic compound that produces a decomposition reaction which generates H₂S, the method, comprising: (a) first adding the hydrolase to the sample and carrying out a reaction which generates H₂S, and then reacting the resulting H₂S with a salt of Cd⁺², thereby fluorescent CdS quantum dots are obtained; and (b) detecting the fluorescent CdS quantum dots of step a). The present invention also comprises said method wherein the detection of fluorescence indicates the detection of the substrate in the sample.

It is still an object of the present invention a method for quantifying the substrate of a hydrolase in a sample, wherein the hydrolase catalyses a hydrolysis reaction of the substrate which yields either H₂S or a thiol-containing organic compound that produces a decomposition reaction which generates H₂S, the method, comprising: (a) first adding the substrate to the sample and carrying out a reaction which generates H₂S, and then reacting the resulting H₂S with a salt of Cd⁺², thereby fluorescent CdS quantum dots are obtained; (b) measuring the fluorescence generated by the CdS quantum dots; (c) generating a calibration curve; and (d) extrapolating the concentration of the substrate in the sample using the calibration curve and the fluorescence measurement obtained for the sample.

The present invention, based on the enzymatic generation of fluorescent CdS QDs is, hence, particularly advantageous in the measurement of the enzymatic activity of hydrolases, such as ALP and AChE, in a wide number of bioanalytical assays. For instance, ALP finds application in ELISA, detection of low levels of ALP in whole blood without separation of the serum, which is compulsory in case of using chromogenic substrates, or the quality control of the pasteurisation process of the cow's milk. AChE finds application in the treatment of Alzheimer's disease or other neurological disorders.

It is also an object of the present invention a method for the preparation of fluorescent CdS quantum dots, the method, comprising first carrying out an enzymatic reaction catalysed by a hydrolase wherein said hydrolase catalyses the hydrolysis reaction of a substrate which yields either H₂S or a thiol-containing organic compound that produces a decomposition reaction which generates H₂S, and then reacting the resulting H₂S with a salt of Cd⁺², thereby fluorescent CdS quantum dots are obtained.

This is the first time that hydrolases are used to efficiently prepare fluorescent CdS QDs.

### BRIEF DESCRIPTION OF THE DRAWINGS

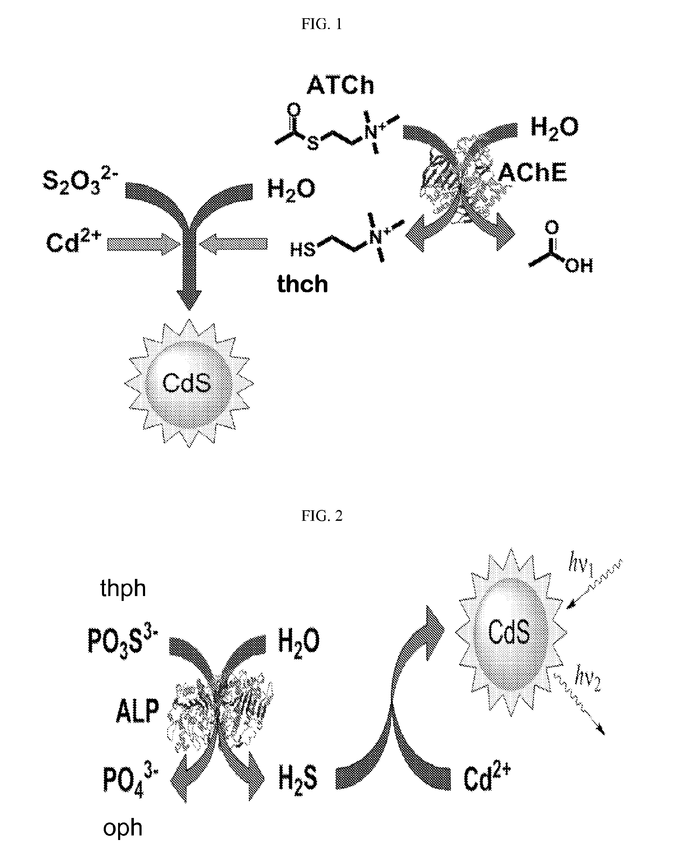

FIG. 1 Scheme of the enzymatic generation of fluorescent CdS QDs for the detection of AChE activity (thch= thiocholine).
FIG. 2 Scheme of the enzymatic generation of fluorescent CdS QDs for the detection of ALP activity (thph= thiophosphate; oph= orthophosphate)

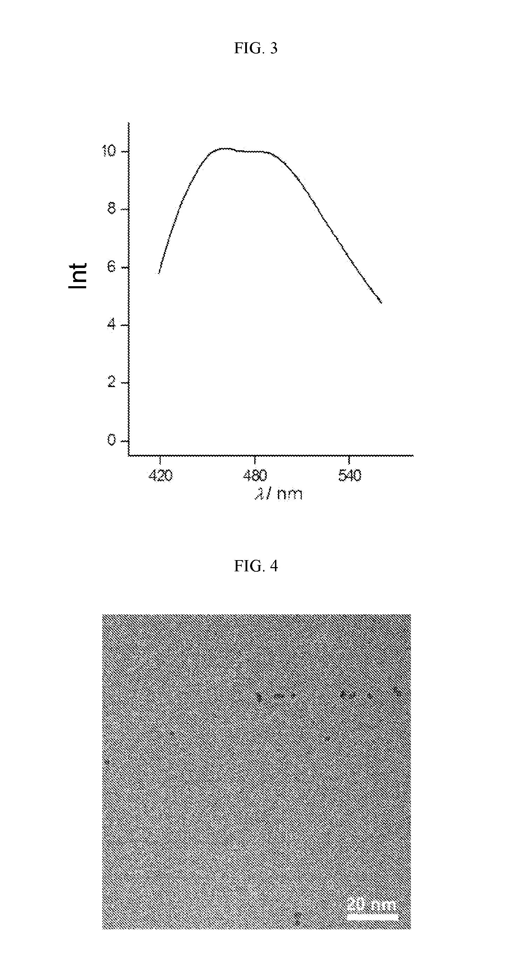

FIG. 3 Emission spectra of CdS QDs formed in the presence of ALP (228 ng mL⁻¹), Na₃SPO₃ (75 µM) and Cd(NO₃)₂ (2.5 mM). The CdS QDs are excited at 360 nm resulting in an emission spectra characterised by a broad peak with a maximum emission at about 445 nm (Int= fluorescence intensity).
FIG. 4 TEM images of fluorescent CdS QDs formed in the presence of ALP (228 ng mL⁻¹), Na₃SPO₃ (75 µM) and Cd(NO₃)₂ (2.5 mM). The formed QDs have a spherical shape with a diameter calculated to be between 1 and 3 nm, with a median diameter of 1.9 nm.

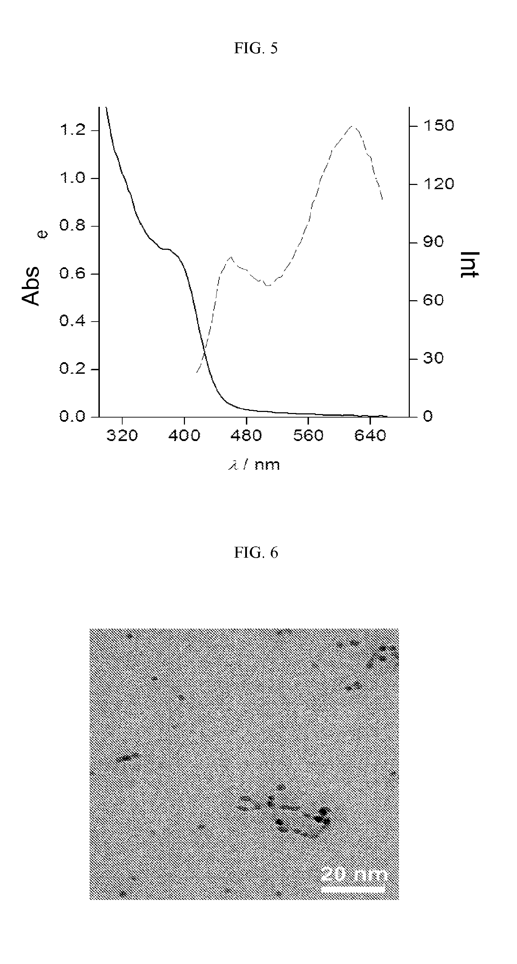

FIG. 5 UV-visible absorption (solid line) and emission (dashed line) spectra of CdS QDs produced by the enzymatic hydrolysis of acetylthiocholine chloride (ATCh). CdS QDs were formed in the presence of ATCh (16 mM), AChE (25 U mL⁻¹), Na₂S₂O₃ (0.3 M) and CdSO₄ (1 mM). From the UV-visible spectrum an increased absorption below 480 nm, and a shoulder at about 380 nm was observed. The shoulder is explained by 1Sₕ - 1Sₑ excitonic transition characteristic of nanoparticles with a diameter of ~2-3 nm. From the emission spectrum of enzymatically generated CdS QDs, a maximum at 600 nm and a lower peak at 440 nm were observed (Abs= Absorbance; Int= Intensity).
FIG. 6 TEM image of the fluorescent CdS QDs formed in the presence of ATCh (16 mM), AChE (10 U mL⁻¹), Na₂S₂O₃ (0.3 M) and CdSO₄ (1 mM). The diameter of the QDs was calculated to be between 2 and 3 nm.
FIG. 7 Calibration curve for the quantification of the ALP present in a sample, based on the representation of fluorescence intensity (Int) at λ=445 nm *versus* the concentration of ALP in ng mL⁻¹.
FIG. 8 Calibration curve for the quantification of the sodium thiophosphate (Na-thph) present in a sample, based on the representation of fluorescence intensity (Int) at λ=445 nm *versus* the concentration of sodium thiophosphate in µM.
FIG. 9 Evolution of the fluorescence intensity (Int) of the CdS QDs generated in the presence of ATCh (10 mM) and different concentrations of AChE: a) 0 mU mL⁻¹; b) 25 mU mL⁻¹; c) 50 mU mL⁻¹; d) 100 mU mL⁻¹ and e) 250 mU mL⁻¹. In all experiments, the system included Na₂S₂O₃ (0.3 M) and CdSO₄ (1 mM).
FIG. 10 Evolution of CdS QDs formation rate with time in the presence of ATCh (10 mM) and different concentrations of AChE a) 0 mU mL⁻¹; b) 25 mU mL⁻¹; c) 50 mU mL⁻¹; d) 100 mU mL⁻¹; and e) 250 mU mL⁻¹. The plot represents the first derivative of fluorescence intensity with respect to time (dl/dt) *versus* time in minutes (t / min). In all experiments, the system included sodium thiosulfate (0.3 M) and CdSO₄ (1 mM).
FIG. 11 Calibration curve for the quantification of the AChE present in a sample, based on the representation of the times of maximum autocatalytic rate in minutes (*t* / min) *versus* concentration of AChE in mU mL⁻¹ (AChE / mU mL⁻1).
FIG. 12 Evolution of the fluorescence intensity (Int) of the CdS QDs formed in the presence of AChE (25 U mL⁻¹) and different concentrations of ATCh: a) 8 mM; b) 12 mM; c) 16 mM; d) 20 mM; e) 24 mM; f) 32 mM and g) 40 mM. The plot represents fluorescence intensity (Int) *versus* time in minutes (t / min). In all experiments, the system included sodium thiosulfate (0.3 M) and CdSO₄ (1 mM).
FIG.13 Evolution of CdS QDs formation rate with time in the presence of AChE (25 U mL⁻¹) and different concentrations of ATCh: a) 8 mM; b) 12 mM; c) 16 mM; d) 20 mM; e) 24 mM; f) 32 mM and g) 40 mM. The plot represents the first derivative of fluorescence intensity with respect to time (dl/dt) *versus* time in minutes (t / min). In all experiments, the system included Na₂S₂O₃ (0.3 M) and CdSO₄ (1 mM).
FIG. 14 Calibration curve for the quantification of ATCh present in a sample, based on the representation of the times of maximum autocatalytic rate in minutes (t / min) *versus* concentration of ATCh in mM (ATCh / mM).

### Definitions

In general, the following words or phrases have the indicated definition when used in the description, examples and claims.

The term "nanoparticle" is known to the person skilled in the art.

The term "quantum dot" is known to the person skilled in the art. The abbreviations QD and QDs are being used in the present invention to refer to quantum dot and quantum dots, respectively.

The terms nanoparticle, nano-particle, quantum dot, nano (size) particle, nanocrystal and QD have the same meaning and are used indistinctly in the present invention.

The term "measuring" according to the present invention relates to determining the concentration or the quantity (or amount), semiquantitatively or quantitatively by making a single measurement, making multiple measurements, or monitoring by making continuous measurements. In addition, the term "measuring" encompasses both detecting the presence of a component and quantifying the concentration or quantity of a component.

The term "amount" refers to either the concentration or the quantity (or amount) (which may be zero or non-zero) of a component in a sample.

The expression ALP (enzymatic) activity, AChE (enzymatic) activity or hydrolase (enzymatic) activity or (enzymatic) activity of ALP, (enzymatic) activity of AChE or (enzymatic) activity of a hydrolase is a measure of the quantity of active ALP, AChE or hydrolase present in a sample expressed either as milliunits of enzyme activity per millilitre of sample (mU/mL or mU mL⁻¹), which is the amount of enzyme required to catalyse the hydrolysis of 1 nmol of substrate per min, or expressed in nanograms per millilitre (ng mL⁻¹).

The term "intensity" refers to the measure of the fluorescence and is expressed in arbitrary units.

The term "substrate" is known to the person skilled in the art. "Substrate" refers to a molecule upon which an enzyme acts. Enzymes, hence, catalyse chemical reactions involving the substrate(s).

### DETAILED DESCRIPTION OF THE INVENTION

Unless specifically defined herein, all terms used herein have the same meaning as they would to one skilled in the art of the present invention.

The present invention overcomes many of the limitations mentioned in a section above. In this regard, the inventors have surprisingly found that some hydrolases can be used to catalyse reactions, in which H₂S is generated, so that said reaction product in the presence of Cd⁺² generates fluorescent CdS QDs.

The hydrolase of the methods of the present invention could be any hydrolase that catalyses a hydrolysis reaction of a substrate which yields either H₂S or a thiol-containing organic compound that produces a decomposition reaction which generates H₂S. Such hydrolases include but are not limited to: paraoxonases (also called PON) (EC 3.1.8.1) a group of enzymes involved in the hydrolysis of organophosphates, the paraoxonases play multifunctional role in metabolism; monoacylglycerol lipase (also called MGLL) (EC 3.1.1.23); thioester hydrolases (EC 3.1.2), which includes hydrolases which act on ester bonds; AChE (EC 3.1.1.7); ALP (EC 3.1.3.1); D-cysteine desulfhydrase (EC 4.4.1.15); cystathionine gamma-lyase (EC 4.4.1.1); homocysteine desulfhydrase (EC 4.4.1.2); and cystathionine beta-synthase (EC 4.2.1.22).

Based on the above unexpected effect, methods for the detection/quantification of the enzymatic activity of some hydrolases and methods for the detection/quantification of their substrates based on the generation of fluorescent CdS quantum dots, as described in the section "Summary of the invention" are part of the underlying invention.

In a preferred embodiment of the above methods the hydrolase catalyses the reaction of a substrate which yields H₂S.

In a more preferred embodiment, the hydrolase is the ALP.

In another preferred embodiment of the present invention the method is based on the synthesis of fluorescent CdS QDs in the presence of ALP, thiophosphate and Cd⁺². In the presence of ALP, thiophosphate is hydrolysed to ortophosphate and H₂S. The latter reacts immediately with a salt of Cd⁺² and gives fluorescent CdS QDs.

In the above method, the fluorescent CdS quantum dots are generated as result of the following reactions:

PO₃S³⁻ + H₂O → PO₄³⁻ + H₂S

H₂S + Cd²⁺ → CdS + 2H⁺

The thiophosphate salt could be any water soluble salt containing thiophosphate anions. In a preferred embodiment the thiophosphate salt is Na₃SPO₃.

The source of Cd⁺² could be any water soluble Cd⁺² salt. In a preferred embodiment, the source of Cd⁺² is Cd(NO₃)₂. In another preferred embodiment, the source of Cd⁺² is CdSO₄.

In another preferred embodiment of the method of the present invention, the hydrolase catalyses a hydrolysis reaction of a substrate which yields a thiol-containing organic compound that produces a decomposition reaction which generates H₂S.

In a preferred embodiment, the hydrolase is AChE.

The substrate could be any compound that yields a thiol-containing organic compound. In a preferred embodiment the substrate is a water soluble salt of acetylthiocholine. In a more preferred embodiment the substrate is the acetylthiocholine chloride (ATCh) and the thiol-containing organic compound is thiocholine.

One embodiment of the present invention is, hence, directed to a method for the detection of AChE activity based on the synthesis of fluorescent CdS QDs in the presence of thiosulfate, Cd⁺² and thiocholine. Anions of thiosulfate, S₂O₃²⁻ are reduced to H₂S which in the presence of Cd²⁺ form nanocrystals of CdS. The rate of the first reaction is slow, but thiol-containing organic compounds promote the decomposition of S₂O₃²⁻ to H₂S. The resulting H₂S reacts with the salt of Cd²⁺ giving fluorescent CdS nanocrystals.

In the above method, the fluorescent CdS quantum dots are generated as result of the following reactions:
Acetylthiocholine chloride + H₂O → thiocholine + acetic acid

S₂O₃²⁻ + H₂O → SO₄²⁻ + H₂S

H₂S + Cd²⁺ → CdS + 2H⁺

The thiosulfate salt could be any water soluble salt containing thiosulfate anions. In a preferred embodiment the thiosulfate salt is Na₂S₂O₃.

The source of Cd⁺² could be any water soluble Cd⁺² salt. In a preferred embodiment, the source of Cd⁺² is Cd(NO₃)₂. In another preferred embodiment the source of Cd⁺² is CdSO₄.

In the methods of the invention, the sample is of any biological tissue or fluid. In another preferred embodiment the sample is milk or a derivative. In yet another preferred embodiment the sample is one to carry out a screening assay. The sample may be a "clinical sample" which is a sample obtained from a patient. Such samples include, but are not limited to, sputum, blood, blood cells (e.g., white cells), tissue or fine needle biopsy samples, urine, peritoneal fluid, and pleural fluid, or cells there from. Biological samples may also include sections of tissues such as frozen sections taken for histological purposes. Consequently, the methods of the invention may be *in vitro* methods. One of the ordinary skilled in the art will treat the sample appropriately depending on the sample type in order to be used in the methods of the present invention without undue experimentation.

As mentioned above, an aspect of the present invention refers to a quantification method wherein the hydrolase enzymatic activity quantified is that of the ALP.

In another embodiment of the present invention the hydrolase substrate quantified is the thiophosphate. The concentrations are determined by extrapolating the value from a calibration plot representing the maximum emission values *versus* different concentrations of ALP or thiophosphate, respectively.

The quantification methods of the present invention may further comprise the preparation of a calibration curve, which indicates the relationship between fluorescence intensity and the concentration of the hydrolase or its substrate, calibration curve which is preferably determined prior to the measurement of the real sample.

The preparation of the calibration curve may comprise preparing several standard solutions containing the hydrolase or substrate at different predetermined concentrations in the conditions and with the reactives that allow the generation of the fluorescent CdS QDs; a measurement step of illuminating the mixed solution with excitation light and obtaining a measurement value by measuring an intensity of fluorescence emitted from the mixed solution; and a calibration curve preparation step of determining a relationship between fluorescence intensity and concentration of the hydrolase or its substrate based on added amounts of the hydrolase or the substrate and the measurement values obtained in the measurement step. By including these steps, a correlation between fluorescence intensity and the concentration of the hydrolase or its substrate, that is, a calibration curve can be obtained efficiently.

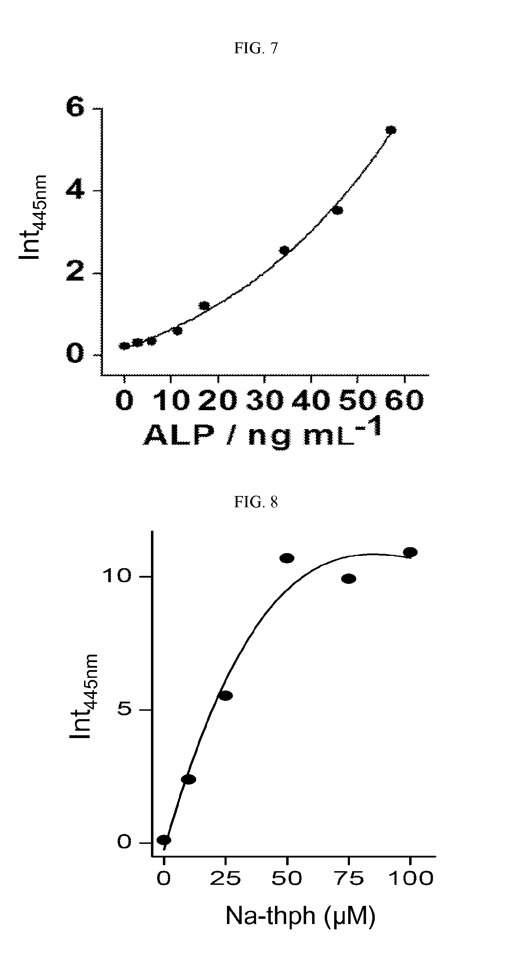

Guidance as to how to extrapolate the concentration of the ALP or thiophosphate present in a sample from a calibration curve as well as obtaining said curve by plotting different ALP or thiophosphate concentrations *versus* the corresponding emission intensity values following the methods of the present invention can be derived from Figures 7 and 8, respectively (the values plotted in the curves are summarised in Tables 1 and 2, respectively).

**Table 1**

| ALP (ng ml⁻¹) | Intensity (Int₄₄₅ₙₘ) |
|---|---|
| 0 | 0,21811 |
| 2,85 | 0,30458 |
| 5,7 | 0,34357 |
| 11,4 | 0,58142 |
| 17,1 | 1,2 |
| 34,2 | 2,55 |
| 45,6 | 3,53 |
| 57 | 5,48 |

**Table 2**

| Na-thph (µM) | Intensity (Int₄₄₅ₙₘ) |
|---|---|
| 100 | 10,9 |
| 75 | 9,92 |
| 50 | 10,7 |
| 25 | 5,53 |
| 10 | 2,38 |
| 0 | 0,109 |

It is evident that the values given there serve as a first reference. The person skilled in the art is able to know how to determine ALP or thiophosphate concentration following the methods of the underlying invention.

Any programme known in the art for plotting calibration curves can be used in the methods of the underlying invention. In a preferred embodiment of the present invention, the programmes used are Origin 8.1 and Excel 2007.

Any fluorometer known in the art for the measurement of fluorescence intensity can be used in the methods of the underlying invention. In a preferred embodiment of the present invention, the fluorescence of the CdS QDs generated by ALP are measured at λ_{exc}=360 nm and λₑₘ=445 nm.

From the calibration plot of Figure 7, and using a S/N (signal to noise ratio)= 3, the detection limit for the quantification of the ALP enzymatic activity is 8 ng mL⁻¹. In contrast to this detection limit, the detection limit for the quantification of the ALP enzymatic activity using the commercial chromogenic substrate p-nitrophenyl phosphate is 35 ng mL⁻¹. In summary, the quantification method of the present invention is four times more sensitive than the commercial assay.

In another preferred embodiment of the present invention the hydrolase quantified is AChE. In another embodiment of the present invention the quantified substrate is ATCh. The quantification method of the invention may further comprise the preparation of a calibration curve, which indicates the relationship between fluorescence intensity and the concentration of the hydrolase or its substrate. The calibration curve is preferably determined prior to the measurement of the sample.

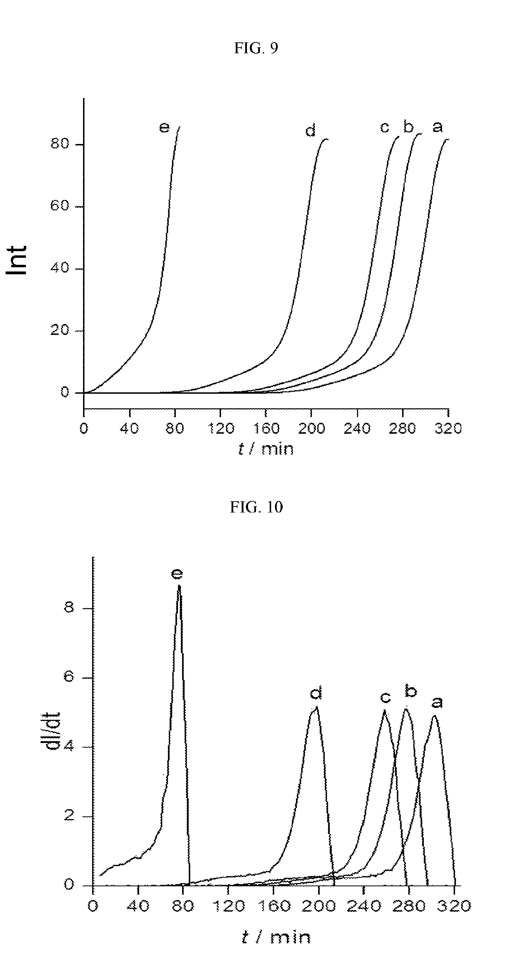

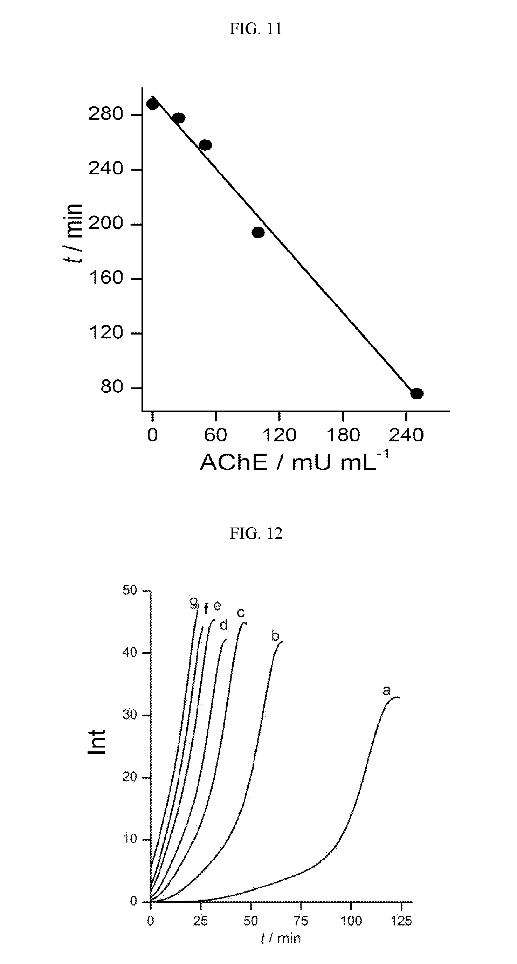

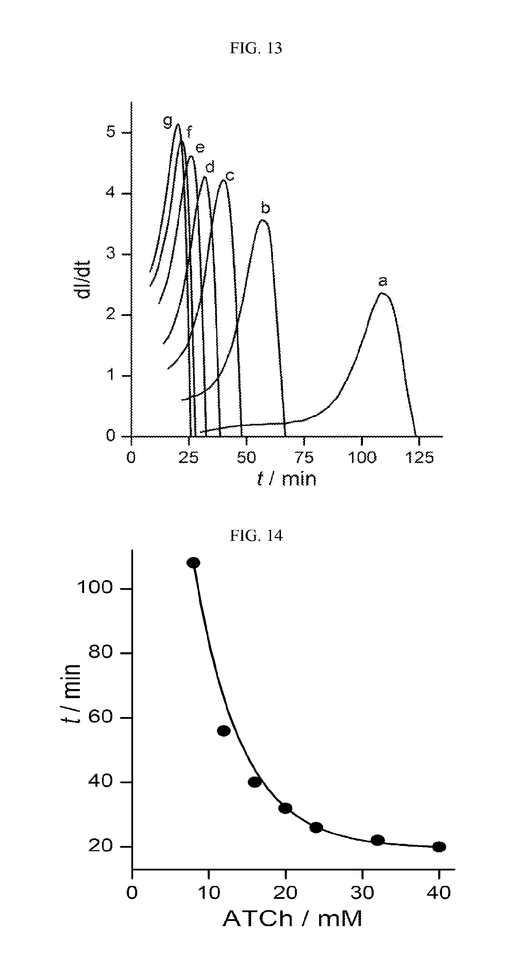

The traditional version of calibration curves described for the quantification of ALP based on the intensity readout of signal measured at fixed time is not suitable in the case of AChE or its substrate, ATCh, because the process of formation of CdS QDs induced by thiocholine is autocatalytic. Consequently, the parameter needed to obtain accurate calibration curves to quantify AChE or ATCh present in a sample, is not the measurement of fluorescence intensities at a fixed time for each concentration of AChE or ATCh but the time of stage of maximum autocatalytic rate, which depends on the concentration of an analyte. The exact times of maximum autocatalytic rates corresponding to each concentration of AChE or ATCh corresponds to the peaks on the curves obtained by plotting the first derivative of fluorescence intensity with respect to time (dl/dt) *versus* time (see Fig. 10 and 13). In a preferred embodiment the measurement of the fluorescence intensity is recorded every 3 minutes. The method further comprises plotting the times of maximum autocatalytic rates versus the concentrations of AChE or ATCh used, and extrapolating from the plotted curved which AChE or ATCh concentration corresponds to the maximum autocatalytic rate obtained for the tested sample. A preferred embodiment of preparing a calibration curve concerning this invention is described in detail in Examples 3 and 4 and Fig 11 and 14 (the values plotted in the curves are summarised in Tables 3 and 4, respectively).

**Table 3**

| AChE (mU ml⁻¹) | time in min (t/min) |
|---|---|
| 0 | 288 |
| 25 | 278 |
| 50 | 258 |
| 100 | 194 |
| 250 | 76 |

**Table 4**

| ATCh (mM) | time in min (t/min) |
|---|---|
| 8 | 108 |
| 12 | 56 |
| 16 | 40 |
| 20 | 32 |
| 24 | 26 |
| 32 | 22 |
| 40 | 20 |

Guidance as to what exact time of maximum autocatalytic rate corresponds to AChE or ATCh concentrations following the methods of the present invention can be derived from those values disclosed in Fig 9, 10, 12 and 13. It is evident that the values given serve as a first reference. The person skilled in the art is able to know how to determine the exact times of maximum autocatalytic rates which correspond to each concentration of AChE and ATCh to be plotted in the methods of the underlying invention.

Any fluorometer known in the art for the measurement of fluorescence intensity can be used in the methods of the underlying invention. In a preferred embodiment of the present invention the fluorescence of the CdS QDs generated by AChE is measured at λ_{exc}=340 nm and λₑₘ=612 nm.

Any programme known in the art for plotting calibration curves can be used in the methods of the underlying invention. In a preferred embodiment of the present invention, the programmes used are Origin 8.1 and Excel 2007.

The assays of the present invention performed in this manner may also be used for the purposes of identifying inhibitors, enhancers, or other modulators of the enzymatic activity of some hydrolases (for example, molecules which change the substrate-response profile of the hydrolase, or alter the manner in which the hydrolase responds to another regulatory molecule). Such a hydrolase assay may be used in conjunction with high-throughput screening methods, such as robotics, with or without automated injection and transfers, for example, to screen or test chemical libraries for inhibiting, enhancing, or modulatory activities against hydrolases.

For instance, if inhibition of AChE is being measured, or an inhibitor of AChE is being detected, then AChE is supplied, optionally in a fixed amount, and the light emission is inversely related to the degree of inhibition of AChE.

Finally, the present invention also comprises a novel method for the preparation of fluorescent CdS quantum dots, the method, comprising first carrying out an hydrolase catalysed enzymatic reaction wherein the hydrolase catalyses a hydrolysis reaction of a substrate which yields either H₂S or a thiol-containing organic compound that produces a decomposition reaction which generates H₂S, and then reacting the resulting H₂S with a salt of Cd⁺², thereby fluorescent CdS quantum dots are generated.

Throughout the description and claims the word "comprise" and variations of the word, are not intended to exclude other technical features, additives, components, or steps. Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular and preferred embodiments described herein.

### EXAMPLES

### Example 1. Generation of QDs by ALP

Sodium thiophosphate (75 µM) was incubated with ALP (228 ng mL⁻¹) in Tris-HCl buffer (0.05 M, pH 8.8) containing MgCl₂ (1 mM) at 37 °C for 90 minutes. After that, 5 µL of Cd(NO₃)₂ (0.5 M) were added to 995 µL of this mixture and incubated at room temperature for 30 min. ALP (from *Escherichia coli*), sodium thiophosphate (Na₃SPO₃) and cadmium nitrate Cd(NO₃)₂ were obtained from Sigma-Aldrich. The emission spectra of the resulting suspension was recorded in a FluoroLog (Horiba Jobin Yvon) fluorometer at λ_{exc}=360 nm. The generated CdS QDs were dialyzed against deionized water and stored at -80°C.

### Example 2. Generation of QDs by AChE

Acetylthiocholine chloride (16 mM) was incubated with AChE (10 U mL⁻¹) in HEPES buffer (0.01 M, pH 8.0) at 37 °C for 90 minutes. Acetylthiocholine chloride, AChE (from electric eel) and cadmium sulfate were obtained from Sigma-Aldrich. The mixture (100 µL) was added to a solution (100 µL) consisting of CdSO₄ (2 mM) and sodium thiosulfate (0.6 M) in water and incubated at room temperature during 60 minutes. The fluorescence of the resulting solution was recorded in a FluoroLog (Horiba Jobin Yvon) fluorometer at λ_{exc}=340 nm. The generated CdS QDs were dialyzed against deionized water and stored at -80°C.

### Example 3. Quantification of ALP

Varying amounts of NaₛSPO₃ were incubated with varying amounts of ALP in Tris-HCl (0.05 M, pH 8.8) containing MgCl₂ (1 mM) at 37 °C for 90 min. After that, 5 µL of Cd(NO₃)₂ (0.5 M) were added to 995 µL of this mixture. ALP (from *Escherichia coli*), Na₃SPO₃ and Cd(NO₃)₂ were obtained from Sigma-Aldrich. The emission intensity of the resulting suspensions was recorded in a FluoroLog (Horiba Jobin Yvon) fluorometer at λ_{exc}=360 nm and λₑₘ=445 nm. The calibration curves were plotted using the programmes Origin 8.1 and Excel 2007.

### Example 4. Quantification of AChE

Varying amounts of acetylthiocholine chloride were incubated with varying amounts of AChE in HEPES buffer (0.01 M, pH 8.0) at 37 °C for 90 minutes. The mixture (100 µL) was added to a solution (100 µL) consisting of CdSO₄ (2 mM) and sodium thiosulfate (0.6 M) in water. Acetylthiocholine chloride, AChE (from electric eel) and CdSO₄ were obtained from Sigma-Aldrich. The fluorescence of the resulting solutions were recorded in a Plate Reader GeniosPro (Tecan) fluorometer at λ_{exc}=340 nm and λₑₘ=612 nm. The calibration curves were plotted using the programmes Origin 8.1 and Excel 2007.

### REFERENCES CITED IN THE APPLICATION

Goldman et al., "A hybrid quantum dot-antibody fragment fluorescence resonance energy transfer-based TNT sensor" J. Am. Chem. Soc. (2005) vol: 127: 6744-6751.
Xiao et al., "Shape and color of Au nanoparticles follow biocatalytic processes" Langmuir (2005) vol. 21: 5659-5662.
Ansary et al., "CdS quantum dots: enzyme mediated in vitro synthesis, characterization and conjugation with plant lectins" J. Biomed. Nanotechnol. (2007) vol. 3: 406-413.
Medintz et al., "Proteolytic activity monitored by fluorescence resonance energy transfer through quantum-dot-peptide conjugates" Nat. Mater. (2006) vol. 5: 581-589.
Horwitz et al., "Substrates for cytochemical demonstration of enzyme activity. II. Some dihalo-3-indolyl phosphates and sulfates" J. Med. Chem. (1966) vol. 9: 447-447.
Mukherjee et al., "Application of a thiol-specific electrocatalytic electrode for real-time amperometric monitoring of enzymatic hydrolisis", Analyst (2009) vol. 134: 582-586.

## Claims

1. A method for detecting enzymatic activity of a hydrolase in a sample wherein the hydrolase catalyses a hydrolysis reaction of a substrate which yields either H₂S or a thiol-containing organic compound that produces a decomposition reaction which generates H₂S, the method, comprising: (a) first adding the substrate to the sample and carrying out a reaction which generates H₂S, and then reacting the resulting H₂S with a salt of Cd⁺², thereby fluorescent CdS quantum dots are obtained; and (b) detecting the fluorescent CdS quantum dots of step a).

2. A method for quantifying enzymatic activity of a hydrolase in a sample, wherein the hydrolase catalyses a hydrolysis reaction of a substrate which yields either H₂S or a thiol-containing organic compound that produces a decomposition reaction which generates H₂S, the method, comprising: (a) first adding the substrate to the sample and carrying out a reaction which generates H₂S, and then reacting the resulting H₂S with a salt of Cd⁺², thereby fluorescent CdS quantum dots are obtained; (b) measuring the fluorescence generated by the CdS quantum dots; (c) generating a calibration curve; and (d) extrapolating the concentration of the hydrolase in the sample using the calibration curve and the fluorescence measurement obtained for the sample.

3. A method for detecting the substrate of a hydrolase in a sample, wherein the hydrolase catalyses a hydrolysis reaction of the substrate which yields either H₂S or a thiol-containing organic compound that produces a decomposition reaction which generates H₂S, the method, comprising: (a) first adding the substrate to the sample and carrying out a reaction which generates H₂S, and then reacting the resulting H₂S with a salt of Cd⁺², thereby fluorescent CdS quantum dots are obtained; and (b) detecting the fluorescent CdS quantum dots of step a).

4. A method for quantifying the substrate of a hydrolase in a sample, wherein the hydrolase catalyses a hydrolysis reaction of the substrate which yields either H₂S or a thiol-containing organic compound that produces a decomposition reaction which generates H₂S, the method, comprising: (a) first adding the substrate to the sample and carrying out a reaction which generates H₂S, and then reacting the resulting H₂S with a salt of Cd⁺², thereby fluorescent CdS quantum dots are obtained; (b) measuring the fluorescence generated by the CdS quantum dots; (c) generating a calibration curve; and (d) extrapolating the concentration of the substrate in the sample using the calibration curve and the fluorescence measurement obtained for the sample.

5. The method according to any of the claims 1-4, wherein the hydrolase catalyses a hydrolysis reaction of a substrate which yields H₂S.

6. The method according to claim 5, wherein the hydrolase is the alkaline phosphatase.

7. The method according to claim 6, wherein the fluorescent CdS quantum dots are generated as result of the following reactions:
PO₃S³⁻ + H₂O → PO₄³⁻ + H₂S
H₂S + Cd²⁺ → CdS + 2H⁺

8. The method according to any of the claims 1-4, wherein the hydrolase catalyses a hydrolysis reaction of a substrate which yields a thiol-containing organic compound which is S₂O₃²⁻ that produces a decomposition reaction which generates H₂S.

9. The method according to claim 8, wherein the hydrolase is the acetylcholine esterase.

10. The method according to claim 9, wherein the thiol-containing organic compound is thiocholine.

11. The method of any of the claims 1-10, wherein the salt of Cd⁺² is a water soluble Cd⁺² salt.

12. The method of claim 11, wherein the salt of Cd⁺² is selected from Cd(NO₃)₂ and CdSO₄.

13. The method according to any of the claims 2, 4, 9 and 10, wherein the calibration curve is obtained by plotting times of maximum autocatalytic rates *versus* concentrations.

14. A method for the preparation of fluorescent CdS quantum dots, the method, comprising first carrying out an enzymatic reaction catalysed by a hydrolase wherein said hydrolase catalyses the hydrolysis reaction of a substrate which yields either H₂S or a thiol-containing organic compound that produces a decomposition reaction which generates H₂S, and then reacting the resulting H₂S with a salt of Cd⁺², thereby fluorescent CdS quantum dots are obtained.
